(19) **Europäisches Patentamt**
European Patent Office
Office européen des brevets

(11) **EP 1 910 808 B1**

(12) **EUROPÄISCHE PATENTSCHRIFT**

(45) Veröffentlichungstag und Bekanntmachung des Hinweises auf die Patenterteilung:
**09.06.2010 Patentblatt 2010/23**

(21) Anmeldenummer: **06753271.3**

(22) Anmeldetag: **14.06.2006**

(51) Int Cl.:
***G01N 21/64*** *(2006.01)* ***A61B 5/00*** *(2006.01)*

(86) Internationale Anmeldenummer:
**PCT/DE2006/001031**

(87) Internationale Veröffentlichungsnummer:
**WO 2007/012301 (01.02.2007 Gazette 2007/05)**

(54) **VERFAHREN SOWIE MESSSYSTEM ZUR ERMITTLUNG DER SAUERSTOFFPARTIALDRUCKVERTEILUNG IN ZUMINDEST EINEM GEWEBEFLÄCHENABSCHNITT, INSBESONDERE HAUTGEWEBEFLÄCHENABSCHNITT**

METHOD AND ALSO MEASUREMENT SYSTEM FOR DETERMINING THE OXYGEN PARTIAL PRESSURE DISTRIBUTION IN AT LEAST ONE TISSUE SURFACE SECTION, IN PARTICULAR SKIN TISSUE SURFACE SECTION

PROCEDE ET SYSTEME DE MESURE POUR DETERMINER LA REPARTITION DE LA PRESSION PARTIELLE EN OXYGENE SUR AU MOINS UNE PARTIE DE LA SURFACE DE TISSUS, NOTAMMENT SUR UNE PARTIE DE LA SURFACE DE TISSUS CUTANES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **29.07.2005 DE 102005036410**

(43) Veröffentlichungstag der Anmeldung:
**16.04.2008 Patentblatt 2008/16**

(73) Patentinhaber: **Biocam GmbH**
**93053 Regensburg (DE)**

(72) Erfinder: **LIEBSCH, Gregor**
**93083 Abertraubling (DE)**

(74) Vertreter: **Glück, Martin et al**
**Postfach 100826**
**93008 Regensburg (DE)**

(56) Entgegenhaltungen:
**US-A- 4 041 932        US-A- 4 476 870**
**US-A- 5 593 899        US-A1- 2003 050 543**
**US-B1- 6 345 191**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 910 808 B1

## Beschreibung

Die Erfindung betrifft ein Verfahren zur Ermittlung der Sauerstoffpartialdruckverteilung in zumindest einem Gewebeflächenabschnitt, insbesondere Hautflächenabschnitt gemäß Patentanspruch 1 sowie ein Messsystem gemäß Patentanspruch 9.

Die Hauptaufgabe von kleinen Gefäßen innerhalb eines Gewebeflächenabschnittes liegt in der Versorgung des Gewebes mit Sauerstoff. Ob diese in ausreichendem Maße erfolgt, kann mittels einer punktuellen Messung des Sauerstoffgehalts an unterschiedlichen Gewebestellen oder einer Messung des Sauerstoffpartialdruckes im Gewebe bestimmt werden. Hierzu sind Verfahren zur punktuellen Messung des Sauerstoffgehalts an unterschiedlichen Gewebestellen bekannt, welche auf Widerstandsmessungen mittels so genannter Clarkelektroden basieren. Messungen des Sauerstoffpartialdruckes in einem Gewebeflächenabschnitt werden mittels so genannter transkutanen Sauerstoffmessungen (TCPO$_2$) durchgeführt. Die Messergebnisse ermöglichen beispielsweise eine Aussage über den Therapieerfolg von Behandlungsmaßnahmen oder über den kritischen Gefährdungsgrad einzelner Körperextremitäten.

In bereits bekannten, auf dem Prinzip der transkutanen Sauerstoffmessung basierenden Messsystemen werden fluoreszenz-optische Sensoren verwendet, die als Gassensoren ausgebildet sind und anstelle der absoluten Sauerstoffmolekülmenge den jeweils im zu vermessenden Gewebe vorliegenden Sauerstoffpartialdruck messen. Somit es ist grundsätzlich möglich, den Einfluss von Sauerstoff auf einen fluoreszenz-optischen Sensor anhand des ermittelten Sauerstoff-Partialdrucks zu quantifizieren. Der in der Messumgebung herrschende Umgebungsluftdruck sowie die vorherrschende Temperatur sind bei derartigen Messungen zu berücksichtigen.

Ein als Gassensor ausgebildeter fluoreszenz-optischer Sensor ist beispielsweise als planarer Sauerstoffsensor ausgebildet, der auf den mit einer entsprechenden Emulsion oder spezialfluid vorbehandelten Gewebeabschnitt flächig aufgetragen wird. Hierdurch wird zwischen dem planaren Sauerstoffsensor und der Gewebeoberfläche eine die Gewebeeigenschaften widerspiegelnde Messumgebung geschaffen. Die Fluoreszenzfarbstoffmoleküle des fluoreszenz-oplischen Sensors befinden sich in einem angeregten Zustand und geben bei Zuführung von Anregungslicht entweder ihre Energie in Form von Fluoreszenzemissionslicht an die Umgebung ab oder übertragen diese sozusagen "strahlungslos" auf ein in der Messumgebung vorliegende Sauerstoffmolekül. Der letztgenannte Fall wird auch als "dynamische Fluoreszenz-Löschung" bezeichnet, dessen Löschungsgrad abhängig von dem im Medium vorliegenden Sauerstoffpartialdruck ist.

Das beschriebene Prinzip der dynamischen Fluoreszenz-Löschung wird zur Ermittlung des Sauerstoffpartialdruckes eingesetzt, indem die intensität einer unter Dauerbeleuchtung erzeugten Fluoreszenz sowie dessen Abklingzeit ("Lifetime") ermittelt wird, und zwar mittels Aufnahme eines oder mehrerer Fluoreszenzbilder. Unter der Abklingzeit wird dabei die Zeitdauer verstanden, die der Floureszenzfarbstoff "nachleuchtet", und zwar nachdem das zur Erzeugung der Fluoreszenz eingesetzte Anregungslicht abgeschaltet wurde. Die Fluoreszenzintesität sowie dessen Abklingzeit geben hierbei Aufschluss über den im Gewebe vorliegenden Sauerstoffpartialdruck. So bewirkt beispielsweise eine Zunahme des Sauerstoffpartialdruckes eine Verkürzung der Abklingzeit der Fluoreszenz.

Der Einsatz einer derartigen Fluoreszenzintensitätsmessung zur Bestimmung des Sauerstoffpartialdruckes in einem Gewebeabschnitt weist jedoch gerade auf unebenen Gewebeoberflächen eine Reihe von Nachteilen auf. Aufgrund der Abhängigkeit der Fluoreszenzintensität von der Anzahl der im betrachteten Aufnahmesegment enthaltenen Farbstoffmoleküle, der Intensität des Anregungslicht sowie des im Gewebe vorliegenden Sauerstoffpartialdruckes ergeben sich unterschiedliche, schwer isolierbare Störeinflüsse. Eine Kalibrierung und/ oder Korrektur der Messung wird damit nahezu unmöglich, insbesondere bei einer Realisierung der fluoreszenz-optischen Sensoren in form von Mikropartikeln, bei der eine homogene verteilung der Fluoreszenzfarbstoffmoleküle gerade auf der unebenen Hautfläche nicht gewährleistet werden kann.

Selbst bei Annahme einer ideal homogenen Farbstoffverteilung sowie einer ideal homogenen Anregung des fluoreszenz-optischen Sensors werden aufgrund der Unebenheiten der Gewebeoberfläche Intensitätsmuster erzeugt, die keinesfalls auf Sauerstoffpartialdruck-Änderungen basieren, sondern vielmehr auf virtuell erhöhte Indikatorkonzentrationen in den einzelnen Abschnitten der Gewebeoberfläche zurückzuführen sind. Somit werden mehr Farbstoffmoleküle pro Flächensegment vermessen, was zu einer erhöhten Fluoreszintensität in den jeweiligen Segmenten führt.

Ein weiterer Nachteil der Fluoreszenzintensitätsmessung entsteht durch die Verwendung transparenter fluoreszenz-optischer Sensoren auf optisch heterogenen Oberflächen, die die Lichteinstrahlung örtlich unterschiedlich absorbieren und/oder reflektieren. Dies führt fallweise zu einer verbesserten oder verminderten Anregungseffizienz. Hierbei wird das durch die Oberfläche reflektierte Anregungslicht ein zweites Mal durch den Sensor geleitet, wobei das absorbierte Anregungslicht lediglich den Sensor ein einziges Mal durchläuft. Hierdurch wird ein nicht referenzierbarer Messfehler erzeugt, der auf die Oberflächenbeschaffenheit des Gewebeflächenabschnittes zurückzuführen ist.

Ferner sind Systeme zur Fluoreszenzdiagnostik bekannt, welche sich die Stoffwechselunterschiede beispielsweise im Porphyrin-Stoffwechsel zwischen Zellen in dysplastischen/tumorösen Geweben und Zellen im normalen Gewebe zunutze machen. Beispielsweise ist aus der DE 101 57 575 A1 ein System zur Visualisierung von Fluoreszenzfarbstoffen zur Fluoreszenzdiagnostik bekannt, bei dem mit wenigstens einer Lichtquelle ein Beobachtungsarsenal mit einem Anregungslicht beaufschlagt wird, sowie über einen optischen Detektor die aufgrund des im Gewebe vorhandenen Fluoreszenzfarbstoff erzeugte Fluoreszenzemission gemessen wird. Hierbei wird der optische Detektor von wenigstens einem Kamerasystem zur Generierung eines Normalbildes sowie eines Fluoreszenzbildes des Beobachtungsareals gebildet. Die wenigstens eine Lichtquelle ist als gepulste Lichtquelle ausgebildet, welche im sichtbaren oder Infrarot/UV-Bereich liegt. Für das beschriebene System wird das Fluoreszenzbild und das Normalbild des gesamten Beobachtungsareals unmittelbar nacheinander detektiert, digitalisiert und in einer Rechnereinheit verarbeitet. Hierdurch entsteht die Möglichkeit, beide Bilder unmittelbar nebeneinander oder aber übereinander auf einem Bildschirm darzustellen und dadurch erkrankte Gewebeareale ohne die im Farb- bzw. Normalbild enthaltenen Informationen zu verlieren. Hierdurch wird eine verbesserte und eindeutigere Fluoreszenzdiagnostik ermöglicht.

Ferner ist aus der US 5,593,899 ein Verfahren und ein System zur Messung des Sauerstoffgehalts zumindest eines Gewebeabschnittes mittels eines fluoreszenz-optischen Sensors bekannt, der auf den zu untersuchenden Gewebeabschnitt aufgebracht wird. Hierbei ist der fluoreszenz-optische Sensor durch eine phosphoreszierende Messschicht gebildet, die auf den zu untersuchenden Gewebeabschnitt aufgetragen und mit einer Sauerstoff-undurchlässigen Folie abgedeckt wird. Ferner wird mittels einer Anregungslichtquelle Anregungslicht erzeugt, welches über eine optische Faser an die phosphoreszierende Messschicht geführt und diese zur Erzeugung einer Fluoreszenz beaufschlagt wird. Mittels eines Detektorsystems, welches aus einer Photodiode, einem daran anschließenden Interferenzfilter und einer transparenten Abdeckung besteht, wird ein von der erzeugten Fluoreszenz abhängiges elektrisches Messsignal erzeugt, das mittels einer A/D-Wandlereinheit oder einer "Sample and Hold"-Einheit in ein digitales Messsignal umgesetzt wird. Hierdurch wird die Fluoreszenzintensität zu unterschiedlichen Zeitpunkten innerhalb der Abklingphase bestimmt und der Sauerstoffdruck ermittelt.

Die US 2003/0050543 A1 und US 6,345,191 B1 offenbaren die Verwendung einer CCD-Kamera zur Aufnahme von Fluoreszenzbildern.

Aus der US 4,476,870 ist eine faseroptische Messsonde bekannt, welche in die Blutgefäße der zu untersuchenden Person eingeführt wird.

Aufgabe der Erfindung ist es, ein Verfahren sowie ein Messsystem aufzuzeigen, mit dem die Ermittlung flächiger Sauerstoffpartialdruckverteilungen in zumindest einem Gewebeflächenabschnitt möglich ist. Zur Lösung dieser Aufgabe ist ein Verfahren gemäß Patentanspruch 1 sowie ein Messsystem gemäß Patentanspruch 9 ausgebildet.

Der wesentliche Aspekt des erfindungsgemäßen Verfahrens zur Ermittlung der Sauerstoffpartialdruckverteilung in zumindest einem Gewebeflächenabschnitt, insbesondere eines Hautgewebeflächenabschnittes ist darin zu sehen, dass ein einen Fluoreszenzfarbstoff aufweisender fluoreszenz-optischer Sensor auf den Gewebeflächenabschnitt aufgebracht wird und der auf dem Gewcbeflächenabschnitt aufgebrachte Fluoreszenzfarbstoff mit Anregungslicht zur Erzeugung einer Fluoreszenz beaufschlagt wird. Hierbei wird in der Anklingphase der Fluoreszenz mittels eines Kamerasystems zumindest ein erstes Fluoreszenzbild und in der Abklingphase der Fluoreszenz zumindest ein zweites Fluoreszenzbild aufgenommen, die Fluoreszenzintensitäten und die Intensitätsintegrale in der An- und Ahklingphase anhand des aufgenommenen ersten und zweiten Fluoreszenzbildes ermittelt und über Verhältnisbildung der ermittelte Intensitätsintegrale die Sauerstoffpartialdruckverteilung in dem zumindest einen Gewebeflächenabschnitt ermittelt. Vorteilhaft wird hierdurch eine flächenmäßige Visualisierung der Sauerstoffverteilung in einem Gewebeflächenabschnitt unter Auswertung der vorliegenden Sauerstoffpartialdruckverteilung auch unter Raumlichtbedingungen möglich. Aufgrund der Verhältnisbildung wird die Messung unabhängig von zu Messfehlern führenden Störeinflüssen und ist durch eine schnelle Ansteuerung des Kamerasystems sowie Auswertung der erhaltenen digitalisierten Bildddaten ebenfalls im "Live-Bildmodus" möglich. Darüber hinaus kann zusätzlich von dem zu vermessenden Gewebeoberflächenabschnitt ein Farbbild erzeugt werden, welches mit dem ersten und/oder zweiten Fluoreszenzbild überlagert oder in Beziehung gebracht wird, um somit eine genauere Lokalisierung beschädigter Gewebearsenale schnell und zuverlässig erkennen zu können.

Weitere vorteilhafte Ausbildungen der Erfindung, insbesondere ein Messsystem zur Ermittlung der Sauerstoffpartialdruckverteilung sind den weiteren Ansprüchen zu entnehmen.

Die Erfindung wird im Folgenden anhand von Figuren an einem Ausführungsbeispiel näher erläutert. Es zeigen:

Fig. 1    eine schematische Darstellung eines Messsystems zur Ermittlung der Sauerstoffpartialdruckverteilung in zumindest einem Gewwbeflächenabschnitt;

Fig. 2    eine Draufsicht auf eine Stirnseite der Messkopfeinheit des Messsystems gemäß Figur 1;

Fig. 3a,b    in einem Diagramm das An- und Abklingverlialten der Fluoreszenz bei unterschiedlichen Sauerstoffpartialdrücken;

Fig. 4a,b    in einem Diagramm die zur Aufnahme des ersten Fluoreszenzbildes in der Anklinkphase vorgesehenen Steuersignale;

Fig. 5a,b    in einem Diagramm die zur Aufnahme des zweiten Fluoreszenzbildes in der Abklingphase vorgesehenen Steuersignale,

Fig. 6      eine Visualisierung der Fluoreszenzintensitätsverfeilung in der Anklingphase anhand einer Bildschirmdarstellung sowie eines Profilplots,

Fig. 7      eine Visualisierung der Fluoreszenzintensitätsverteilung in der Abklingphase anhand einer Bildschirmdarstellung sowie eines Profilplots und

Fig. 8      eine Visualisierung der Verhältnisverteilung anhand einer Bildschirmdarstellung sowie eines Profilplots.

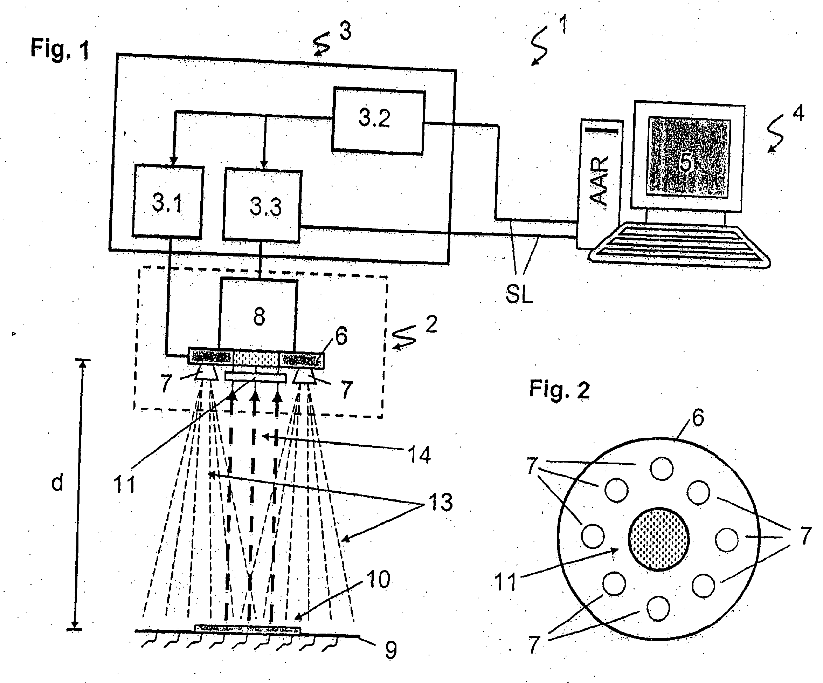

In Figur 1 ist anhand eines schematischen Blockschaltbildes ein Messsystem 1 zur Ermittlung der Sauerstoffpartialdruckverteilung in zumindest einem Gewebeflächenabschnitt dargestellt. Das Messsystem 1 umfasst eine Messkopfeinheit 2, eine Steuereinheit 3 und eine Rechnereinheit 4. Die Steuereinheit 3 ist mit der Messkopfeinheit 2 und der Rechnereinheit 4 verbunden. Zur grafischen Darstellung der Messergebnisse weist die Rechnereinheit 3 ferner zumindest eine Monitoreinheit 5 auf.

Die Messkopfeinheit 2 besteht beispielhaft aus einer Halteplatte 6, die zur Aufnahme von Lichtquellen 7 und zumindest eines Kamerasystems 8 vorgesehen ist. Die Lichtquellen 7 sind vorzugsweise als gepulste Lichtquellen 7 ausgebildet, welche beispielsweise als LED, Blitzlampen, z.B. Xenon-Hochdrucklampen, oder Laserdioden sowie Laserdiodenanordnungen ausgebildet sind.

Auf den zu untersuchenden Gewebeflächenabschnitt 9, vorzugsweise menschlichen oder tierischen Hautgewebeflächenabschnitten wird ein fluoreszenz-optischer Sensor 10 aufgebracht, wobei auf den zu untersuchenden Gewebeflächenabschnitt 9 zuvor beispielsweise eine transparente Emulsion oder Spezialfluid aufgetragen wird, um ein Öffnen der Hautporen zwischen dem Gewebeflächenabschnitt 9 und dem fluoreszenz-optischen Sensor 10 zu bewirken. Hierdurch werden in dem Bereich zwischen dem Gewebeflächenabschnitt 9 und dem fluoreszenz-optischen Sensor 10 optimale Messbedingungen erzeugt. Der fluoreszenz-optische Sensor 10 ist beispielsweise als planarer Sensorfilm oder als Mikropartikelsensor ausgebildet und weist bevorzugt eine rötliche Färbung auf. Ferner ist dieser transparent und besteht aus einer Vielzahl von fluoreszierenden Farbmolekülen. Mittels des fluoreszenz-optischen Sensors 10 wird eine transkutane Sauerstoffmessung nach dem Prinzip der dynamischen Fluoreszenzlöschung realisiert.

Das Kamerasystem 8 ist senkrecht oberhalb des zu vermessenden Gewebeflächenabschnittes 9 in einem Abstand d zwischen 3 - 15 cm angeordnet und weist beispielhaft ein CCD-Modul 11 (Charge-Coupled Device) als opto-elektrischer Bildwandler auf. Ferner ist das Kamerasystem 8 an die Steuereinheit 3 des Messsystems 1 angeschlossen.

In Figur 2 zeigt beispielhaft eine Draufsicht auf die im Betrieb dem Messobjekt zugewandte Stirnseite der Messkopfeinheit 2, in dessen Halteplatte 6 mittig die Linse 12 des CCD-Moduls 11 dargestellt ist. Um die Linse 12 des CCD-Moduls 11 sind mehrere vorzugsweise als LED's realisierte Lichtquellen 7 beispielsweise symmetrisch um die Linse 12 verteilt angeordnet, und zwar derart, dass eine nahezu gleichmäßige Ausleuchtung des zu vermessenden Gewebeflächenabschnittes 9 erzielt wird.

Hierbei ist die zur Erzeugung der Fluoreszenz erforderliche Lichtintensität mittels der Anzahl der Lichtquellen 7 steuerbar. Mehrere derartiger Lichtquellen 7 können zu Lichtquellengruppen (nicht in Figur 2 dargestellt) zusammengefasst werden, welche ebenfalls symmetrisch zur Linse 12 angeordnet sind. In einer bevorzugten Ausführungsform ist eine derartige Lichtquellengruppe mit jeweils ca. 20 -30 LED's ausgestattet. In einer Messkopfeinheit 1 können beispielsweise vier oder mehr von derartigen Lichtquellengruppen vorgesehen sein, so dass insgesamt zumindest zwischen 80 und 120 LED's, vorzugsweise 96 LED's zur Anregung der Fluoreszenz in dem fluoreszenz-optischen Sensor 10 zur Verfügung stehen. Die Anzahl der LED's ist abhängig von der erforderlichen Lichtleistung. Werden beispielsweise LED's mit einer hohen Lichtleistung verwendet, so kann die Anzahl der LED's pro Lichtquellengruppe deutlich reduziert werden.

Die als LED ausgebildeten Lichtquellen 7 sind mittels impulsartiger Ansteuersignale ansteuerbar, welche Schaltflanken im Bereich < = 100 ns ermöglichen. Werden fluoreszenz-optische Sensoren 10 mit Porphyrinen als Floureszenzfarbstoff verwendet, deren maximale Absorption für die Fluoreszenzanregung im blauen Spektralbereich (ca. 405 nm) und deren maximale Fluoreszenzemission im roten Spektralbereich (ca. 630 nm) liegen, erfolgt die Anregung über so genannte "blaue", d.h. im UV-Bereich arbeitende LED's als Lichtquellen 7. Zusätzlich zu den "blauen" LED's können ferner noch "weiße" LED's zur verbesserten Ausleuchtung des zu untersuchenden Gewebeflächenabschnitt 9 während zusätzlicher Farbbildaufnahmen beispielsweise ebenfalls in der Halteplatte 6 vorgesehen sein.

Das von den gepulsten Lichtquellen 7 erzeugte Anregungslicht 13 wird auf die auf dem zu untersuchenden Gewebeflächenabschnitt 9 aufliegenden fluoreszenz-optischen Sensor 10 aufgebracht. Hierbei trifft das Anregungslicht 13 vorzugsweise senkrecht auf den fluoreszenz-optischen Sensor 10 auf. Das von dem fl uoreszenzoptischen Sensor 10 erzeugte Emissionslicht 14 wird über das CCD-Modul 11 des Kamerasystems 8 in der Messkopfeinheit 2 erfasst und in ein elektrisches Bilddatensignal umgewandelt.

Zur Ansteuerung der gepulsten Lichtquelle(n) 7 ist in der Steuereinheit 3 ein Lichtquellenansteuermodul 3.1 vorgesehen, welches mit einer ebenfalls in der Steuereinheit 3 vorgesehenen Triggersignaleinheit 3.2 verbunden ist. Zusätzlich weist die Steuereinheit 3 ein ebenfalls mit der Triggersignaleinheit 3.2 verbundenes Kamerasteuermodul 3.3 auf, dass zur Steuerung des Kamerasystems 8 bzw. des CCD-Moduls 11 vorgesehen ist. Die Triggersignaleinheit 3.2 sowie das Kamerasteuermodul 3.3 sind über Steuerleitungen SL mit der Rechnereinheit 4 verbunden. In der Rechnereinheit 4 ist eine Ansteuer- und Auswerteroutine AAR zur Auswertung von der Steuereinheit 3 empfangenen Messdaten beispielsweise in Form von digitalisierten Bilddaten vorgesehen. Gesteuert über die Ansteuer- und Auswerteroutine AAR wird in der Triggersignaleinheit 3.2 ein rechteckförmiges Triggersignal ts erzeugt, welches gleichzeitig an das Lichtquellenansteuermodul 3.1 und an das Kamerasteuermodul 3.3 geführt wird.

Mittels dem Lichtquellenansteuermodul 3.1 werden die Lichtquellen 7 entsprechend dem Triggersignal ts angesteuert, und zwar derart, dass ein Anregungslichtimpuls einer Länge von ca. 100 $\mu$s erzeugt wird, mit dem der zu untersuchende Gewebeflächenabschnitt 9 beaufschlagt wird. Die Aufnahme von Fluoreszenzbildern erfolgt ebenfalls getriggert über das Triggersignal ts, und zwar mittels des Kamerasteuermoduls 3.3, welches das Kamerasystem 8 bzw. das CCD-Modul 11 entsprechend dem Triggersignal ts ansteuert. In einer bevorzugten Ausführungsform werden neben Fluoreszenzbildern auch Normalbilder (RGB-Aufnahmen) über das Kamerasystem 8 aufgenommen, und zwar abwechselnd mit den Fluoreszenzbildaufnahmen beispielsweise in einem Rhythmus von 2:3, d.h. zunächst werden unmittelbar hintereinander ein erstes und zweites Fluoreszenzbild FB1, FB2 erzeugt, gefolgt von einem ersten bis dritten Normalbild NB1 - NB3.

In einer bevorzugten Ausführungsform stimmt die Aufnahmezeit der Fluoreszenzbilder mit der Anregungszeitdauer überein und beträgt somit ebenfalls ca. 100 $\mu$s. Dagegen kann die Aufnahmezeit für ein Normalbild variabel, vorzugsweise zwischen 5 - 30 ms gewählt werden. Die Bildaufnahmefrequenz der Normalbilder liegt damit beispielsweise bei ca. 33,33 Hz und die der Fluoreszenzbilder demnach bei ca. 6,7 Hz.

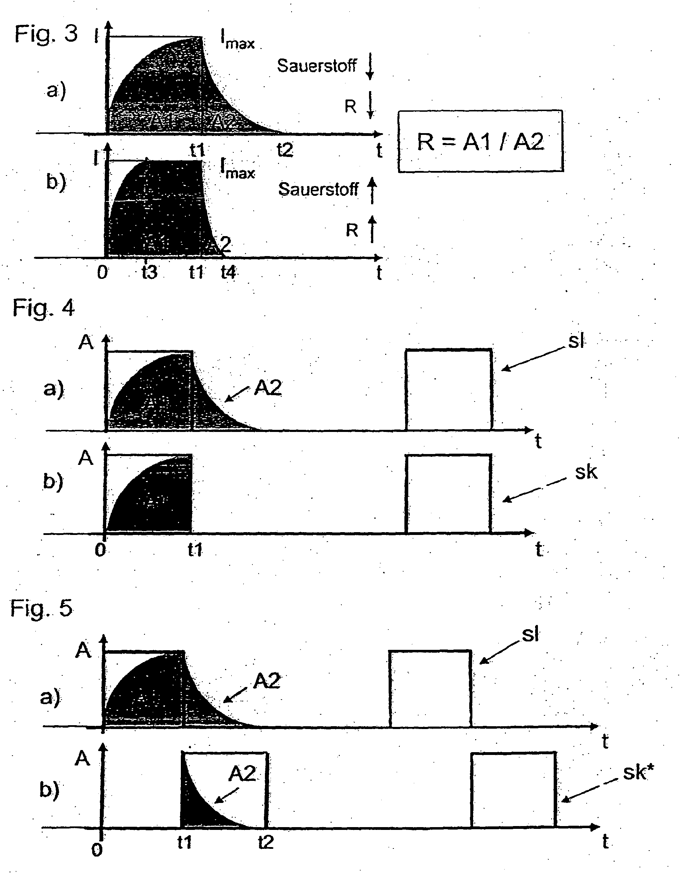

In Fig. 3a und 3b ist beispielhaft der Verlauf der Intensität I des Emissionslichtes 14 bzw. der erzeugten Fluoreszenz bei einem ersten und zweiten Sauerstoffpartialdruck P1, P2 über der Zeit t aufgetragen. Der fluoreszenz-optische Sensor 10 wird hierzu über eine vom Zeitpunkt t = 0 bis zu einem ersten Zeitpunkt t1 sich erstreckende Anregungsdauer mit Anregungslicht 13 beaufschlagt und die Intensität I der Fluoreszenz über der Zeit t ermittelt. Abhängig von der vorliegenden Sauerstoffkonzentration bzw. des ersten und zweiten Sauerstoffpartialdruckes P1, P2 im zu untersuchenden Gewebeflächenabschnitt 9 verkürzt sich die Ankling- und Abklingphase AnP, AbP der Fluoreszenz. Die Anklingphase AnP bezeichnet die von der Anregung der Fluoreszenz bis zum Erreichen eines Sättigungswertes $I_{max}$ erforderliche Zeitdauer und die Abklingphase AbP die nach Abschalten des Anregungslichtes 13 zum Abklingen des

erreichten Sättigungswertes $I_{max}$ bis zum Wert Null erforderliche Zeitdauer.

Bei einer geringeren Sauerstoffkonzentration und somit einem geringen ersten Sauerstoffpartialdruck P1 (Figur 3a) erstreckt sich die Anklingphase AnP über den gesamten Anregungszeitraum bis zum ersten Zeitpunkt t1. Ebenso ergibt sich eine relativ lange Abklingphase AbP, welche sich vom ersten Zeitpunkt t1 bis zu einem zweiten Zeitpunkt t2 erstreckt. In Figur 3b ist der Verlauf der Intensität I des Fluoreszenz für eine erhöhte Sauerstoffkonzentration bzw. einen zweiten Sauerstoffpartialdruck P2 dargestellt, welcher im Vergleich zu dem in Figur 3a dargestellten Verlauf deutlich verkürzte An- und Abklingphasen AnP, AbP aufweist. Die Anklingphase AnP erstreckt sich im betrachteten Ausführungsbeispiel vom ersten Zeitpunkt t=0 bis zu einem dritten Zeitpunkt t3, an dem der Sättigungswertes $I_{max}$ erreicht ist und behält den erreichten Sättigungswertes $I_{max}$ bis einschließlich zum ersten Zeitpunkt t1 bei, an dem das Anregungslicht 13 abgeschaltet wird. Nach Abschalten des Anregungslichtes 13 zum ersten Zeitpunkt t1 beginnt die Abklingphase AbP und erstreckt sich bis zu einem vierten Zeitpunkt t4, der deutlich früher als der zweite Zeitpunkt t2 eintritt.

Durch Bildung eines ersten Intensitätsintegrals A1 über den Anregungszeitraum von t=0 bis t=t1 und eines zweiten Intensitätsintegrals A2 über die Abklingphase AbP bzw. den Abklingzeitraum von t=t1 bis t=t2 bzw. t3 kann das von der vorliegenden Sauerstoffpartialdruckverteilung abhängige Fluoreszenzverhalten quantifiziert werden. Hierzu werden aus den digitalisierten Bilddaten des ersten und zweiten Fluoreszenzbildes FB1, FB2 das erste und zweite Intensitätsintegrals A1, A2 gewonnen und zur Beseitigung von Störeinflüssen das folgende Verhältnis R aus dem gewonnen ersten und zweiten Intensitätsintegralen A1, A2 ermittelt:

$$R = A1 / A2$$

Das ermittelte Verhältnis R weist eine direkte Proportionalität zur Sauerstoffpartialdruckverteilung im zu untersuchenden Gewebeflächenabschnitt 9 und eine umgekehrte Proportionalität zur Abklingdauer der Fluoreszenz auf.

In Figur 4a ist ein zur Ansteuerung der Lichtquellen 7 vorgesehenes erstes Steuersignal sl und in Figur 4b ein zur Ansteuerung des Kamerasystems 8 vorgesehenes zweites Steuersignal sk jeweils gegenüber dem Verlauf der Intensität I des Fluoreszenz beispielhaft dargestellt. Das erste und zweite Steuersignal sl, sk sind vorzugsweise als periodische Rechtecksignale mit einer Amplitude A ausgebildet, dessen Phasenlage getriggert über das Triggersignal ts übereinstimmen. Sowohl das erste als auch das zweite Steuersignal sl, sk nehmen zum Zeitpunkt t=0 die Amplitude A an und fallen zum Zeitpunkt t1 auf den Wert Null zurück. Durch das zweite Steuersignal sk wird die Aufnahme des Kamerasystems 8 gesteuert und ein erstes Fluoreszenzbildes FB1 in der Anklingphase AnP der Fluoreszenz erzeugt.

Zur Aufnahme eines zweiten Fluoreszenzbildes FB2 in der Abklingphase AbP der Fluoreszenz wird die Phase des in Figur 4b dargestellten zweite Steuersignal sk um eine exakt der Anregungsdauer entsprechende Phasenverschiebung von ca. 100 μs verschoben. Somit ergibt sich das in Figur 5b dargestellte dritte Steuersignal sk* zur Ansteuerung des Kamerasystems 8 zur Aufnahme des zweiten Fluoreszenzbildes FB2 in der Abklingphase AbP. Das dritte Steuersignal sk* ist ebenfalls als periodisches Rechtecksignal mit einer Amplitude A ausgebildet. In Figur 5a ist nochmals das zur Ansteuerung der Lichtquellen 7 vorgesehene erste Steuersignal sl dargestellt. Aufgrund der der Anregungsdauer entsprechenden Phasenverschiebung um ca. 100 μs zwischen dem ersten und dritten Steuersignal sl, sk* wird mittels dem zum ersten Zeitpunkt t1 seine Amplitude A annehmenden dritten Steuersignal sk* die Aufnahme des zweiten Fluoreszenzbildes FB2 gestartet und somit die gesamte Abklingphase AbP der Fluoreszenz bis zum zweiten Zeitpunkt t2 aufgezeichnet.

Die mittels der dargestellten ersten bis dritten Steuersignals sl, sk, sk* erzeugten ersten und zweiten Fluoreszenzbilder FB1, FB2 werden von dem Kamerasteuermodul 3.3 an die Rechnereinheit 4 übertragen und in dieser mittels der Ansteuer- und Auswerteroutine AAR ausgewertet. Zur Ermittlung der Fluoreszenzintensitäten werden anhand der beispielsweise in digitaler Form vorliegenden Bilddaten des ersten und zweiten Fluoreszenzbildes FB1, FB2 das erste und zweite Intensitätsintegral A1, A2 ermittelt und anschließend deren Verhältnis R berechnet.

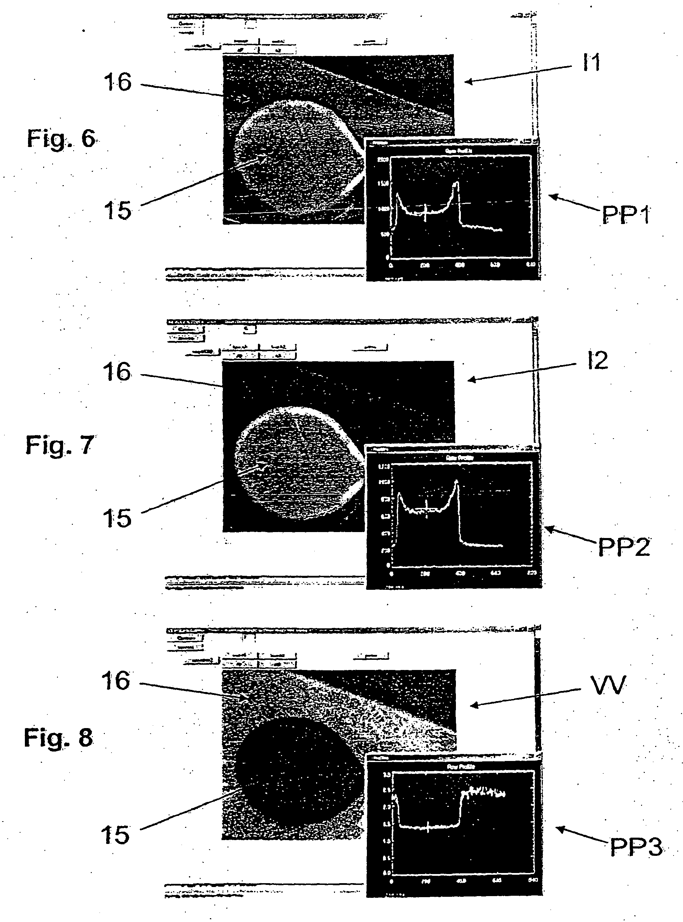

Im Folgenden wird beispielhaft mittels des erfindungsgemäßen Verfahrens die Sauerstoffpartialdruckverteilung eines Tropfens 15 wässrigen Natrium-Sulfit-Lösung unter Umgebungsluft ermittelt, der auf einer Tischoberfläche aufliegt. Die ermittelten Fluoreszenzintensitätsverteilung sind in den Figuren 6 bis 8 jeweils anhand einer Monitordarstellung sowie eines Profiplots PP1 bis PP3 dargestellt. Hierzu wird ein planarer Sauerstoffsensor 16 als fluoreszenz-optischer Sensor 10 verwendet. Der Tropfen 15 ist im Gegensatz zur Umgebungsluft quasi sauerstofffrei, da der in der wässrigen Lösung enthaltene Sauerstoff in Form von Sulfit ($SO_3^{2-}$) zu Sulfat ($SO_4^{2-}$) oxidiert und dadurch aus der wässrigen Lösung entweicht. Der Sauerstoffpartialdruckunterschied zwischen Tropfen 15 und Umgebungsluft wird durch eine veränderte Fluoreszenzintensität und das zugehörige Abklingverhalten wiedergegeben. Die Bildaufnahme erfolgte hierbei unter Raumlichtbedingungen.

Gemäß dem zuvor beschriebenen Verfahren wurde ein erstes und zweites Fluoreszenzbild FB1, FB2 erzeugt und an die Rechnereinheit 4 übertragen und mittels der Ansteuer- und Auswerteroutine AAR jeweils anhand der in Form von digitalisierten Bilddaten vorliegenden ersten und zweiten Fluoreszenzbilder FB1, FB2 die Fluoreszenzintensität I1, I2 in der An- und Abklingphase AnP, AbP der Fluoreszenz sowie deren Verhältnis R ermittelt.

In Figur 6 ist beispielsweise mittels einer Grauwertdarstellung die Fluoreszenzintensitätsverteilung I1 in der Anklingphase AnP der Fluoreszenz des gesamten Messbereichs und in einem ersten Profilplot PP1 entlang einer ausgewählten Schnittebene durch den Messbereich dargestellt. Hierbei zeigt der erste Profilplot PP1 die gemessen Grauwerte einer horizontalen Datenreihe aus dem mittleren Bereich der Fluoreszenzintensitätsverteilung 11. Die Fluoreszenzintensitätsverteilung 11 weist deutliche Inhomogenitäten (z.B. der "11 Uhr - Strich" innerhalb des Tropfens 15) auf. Die Ränder des Tropfens 15 weisen ferner eine höhere Intensität auf, welche auch durch zwei Spitzen im ersten Profilplot PP1 ersichtlich ist. Diese ist jedoch nicht auf einen niedrigeren Sauerstoffpartialdruck zurückzuführen, sondern durch die Lichtleitung innerhalb des Tropfens 15 sowie eine effizientere Auskopplung des Lichtes an den Tropfenrändern bedingt.

In Figur 7 ist die während der Abklingphase AbP gemessene Fluoreszenzintensitätsverteilung I2 mit zugehörigem zweiten Profilplot PP2 dargestellt. Prinzipiell weist die Fluoreszenzintensitätsverteilung I2 dieselben Strukturen wie zuvor auf, d.h. Inhomogenitäten (z.B. der "11 Uhr - Strich" innerhalb des Tropfens 15) des planaren Sauerstoffsensors 16 sind auch in Figur 7 weiterhin zu erkennen, jedoch mit unterschiedlichen Intensitäten.

In Figur 8 ist das Verhältnis R der beiden Fluoreszenzintensitätsverteilung I1, I2 anhand einer Verhältnisverteilung VV dargestellt. Aufgrund der Verhältnisbildung sind nunmehr sämtlichen Inhomogenitäten wie beispielsweise der genannte "11 Uhr-Strich" und die Maxima an den Tropfenrändern verschwunden. Das aufgrund der Störeffekte hervorgerufene Rauschen des planaren Sauerstoffsensors 16 ist innerhalb des einen geringen Sauerstoffpartialdruck aufweisenden Tropfens 15 deutlich reduziert im Vergleich zum höheren Sauerstoffpartialdruck in der Tropfenumgebung. Das beschriebene Verhalten des fluoreszenz-optischen Sensors 10 ist insbesondere für medizinische Anwendungen geeignet, da die dort anzutreffenden Sauerstoffpartialdruckverteilungen häufig einen äußerst geringen Wert aufweisen. Auch die im dritten Profiplot PP3 dargestellten Grauwerte einer horizontalen Datenreihe durch die Verhältnisverteilung VV, insbesondere die fehlenden Spitzen an den Tropfenränder, zeigen eine deutliche Reduzierung der Störeffekte.

Durch die Aufnahme zusätzlicher Normalbilder NB1 - NB3 und die Überlagerung dieser mit dem ersten und zweiten Fluoreszenzbilder FB1, FB2 bzw. deren Fluoreszenzintensitätsverteilungen I1, I2 und/oder mit der Verhältnisverteilung VV kann somit die Sauerstoffverteilung in einem Gewebeflächenabschnitt 9 zusammen mit dem Farbbild über die Monitoreinheit 5 visualisiert werden, wodurch beispielsweise einem Arzt die Identifizierung von eine zu geringe Sauerstoffkonzentration aufweisenden Bereichen des untersuchten Gewebeflächenabschnittes deutlich erleichtert.

Die Bildaufnahme erfolgt hierbei bevorzugt unter Raumlichtbedingungen und/oder in einem Live-Modus.

Die Erfindung wurde voranstehend an einem Ausführungsbeispiel beschrieben. Es versteht sich, dass zahlreiche Änderungen sowie Modifikationen möglich sind, ohne das dadurch der der Erfindung zugrunde liegende Erfindungsgedanke verlassen wird.

**Bezugszeichenliste**

| | |
|---|---|
| 1 | Messsystem |
| 2 | Messkopfeinheit |
| 3 | Steuereinheit |
| 3.1 | Lichtquellenansteuermodul |
| 3.2 | Triggersignaleinheit |
| 3.3 | Kamerasteuermodul |
| 4 | Rechnereinheit |
| 5 | Monitoreinheit |
| 6 | Halteplatte |
| 7 | Lichtquelle(n) |
| 8 | Kamerasystem |
| 9 | Gewebeflächenabschnitt |
| 10 | fluoreszenz-optischer Sensor |
| 11 | CCD-Modul |
| 12 | Linse |
| 13 | Anregungslicht |
| 14 | Emissionslicht |
| 15 | Tropfen |
| 16 | planarer Sauerstoffsensor |
| AAR | Ansteuer- und Auswerteroutine |
| A1 | erstes Intensitätsintegral |
| A2 | zweites Intensitätsintegral |
| d | Abstand |
| FB1 | erstes Fluoreszenzbild |
| FB2 | zweites Fluoreszenzbild |
| I | Intensität der Fluoreszenz |
| $I_{max}$ | Sättigungswert |
| I1, I2 | Fluoreszenzintensitätsverteilungen |
| NB1 | erstes Normalbild |
| NB2 | zweites Normalbild |
| NB3 | drittes Normalbild |
| P1 | erster Sauerstoffpartialdruck |
| P2 | zweiter Sauerstoffpartialdruck |
| PP1 | erster Profilplot |
| PP2 | zweiter Profilplot |
| PP3 | dritter Profilplot |
| SL | Steuerleitungen |
| t1-t4 | erster bis vierter Zeitpunkt |
| ts | Triggersignal |
| W | Verhältnisbild |

**Patentansprüche**

1. Verfahren zur Ermittlung der Sauerstoffpartialdruckverteilung in zumindest einem Gewebeflächenabschnitt (9), insbesondere eines Hautgewebeflächenabschnittes,

   - bei dem ein Fluoreszenzfarbstoff aufgebracht wird,
   - bei dem der auf dem Gewebeflächenabschnitt (9) aufgebrachte Fluoreszenzfarbstoff mit Anregungslicht (13) zur Erzeugung einer Fluoreszenz beaufschlagt wird,
   - bei dem in der Anklingphase (AnP) der Fluoreszenz mittels eines Kamerasystems (8) zumindest ein erstes Fluoreszenzblid (FB1) und in der Abklingphase (AbP) der Fluoreszenz zumindest ein zweites Fluoreszenzbild (FB2) aufgenommen werden,
   - bei dem die Fluoreszenzintensitätem und die Intensitätsintegrale (A1, A2) in der An- und Abklingphase (AnP, AbP) anhand der aufgenommenen ersten und zweiten Fluorexzenzbilder (FB1, FB2) bestimmt werden und
   - bei dem über Verhältnisbildung der ermittelten Intensitäitsintegrale (A1, A2) die Sauerstoffpartialdruckverteilung (P1, P2) in dem zumindest einen Gewebeflächenabschnitt (9) ermittelt wird.

2. Verfahren nach Anspruch 1, bei dem zur Anregung der Fluoreszenz gepulstes Anregungslicht (13) verwendet wird, dessen Spektrum vorzugsweise im UV-Bereich liegt.

3. Vorfahren nach Anspruch 2, bei dem das gepulste Anregungslicht (13) mittels einer gepulsten Lichtquelle (7) erzeugt wird.

4. Verfahren nach einem der Ansprüche 1 bis 3, bei dem die Anregungsdauer gleich der Aufnahmedauer gewählt wird, wobei die Anregungsdauer vorzugsweise 100 μs aufweist und/oder bei dem die Aufnahme des ersten und zweiten Fluoreszenzbildes (FB1, FB2) über ein Triggersignal (ts) gesteuert wird und /oder in der An- und Abklingphase (AnP, AbP) die ersten und zweiten Fluoreszenzbilder (FB1, FB2) als digitale Bilddaten erfasst werden und ausgehend davon die Fluoreszenzintensitäton als erstes und zweites Intensitätsintegral (A1, A2) ermittelt wird.

5. Verfahren nach einem der Ansprüche 4, bei dem das Verhältnis (R) des ersten zum zweiten Intensitätsintegral (A1, A2) ermittelt wird, welches ein Maß für die in dem zu untersuchenden Gewebeflächenabschnitt (9) vorliegende Sauerstoffpartialdruckverteilung (P1, P2) ist.

**6.** Verfahren nach einem der vorhergehenden Ansprüche, bei dem zusätzlich zu den aufgenommenen Fluoreszenzbildern (FB1, FB2) über das Kamerasystem (8) Normalbilder (NB1-NB3) des zu untersuchenden Gewebeflächenabschnitts (9) aufgenommen werden und/oder bei dem als Floureszenzfarbstoff Porphyrine verwendet werden.

**7.** Verfahren nach Anspruch 6, bei dem die zur Aufnahme der Normalbilder vorgesehene Aufnahmedauer zwischen 1 - 30 ms gewählt: wird.

**8.** Verfahren nach einem der vorhergehenden Ansprüche, bei dem vor dem Aufbringen des Fluoreszenzfarbstoffes auf den zu untersuchenden Gewebeflächenabschnin (9) eine transparente Emulsion oder ein transparentes Spezialfluid aufgetragen wird und/oder bei dem die Intensität des Anregungslichtes (13) mittels der Anzahl der vorzugsweise als LED ausgebildeten Lichtquellen (7) geregelt wird.

**9.** Messsystem zur Ermittlung der Sauerstoffpartialdruckverteilung in zumindest einem Gewebeflächenabschnitt (9), insbesondere eines Hautgewebeflächenabschnittes,

- mit mindestens einem einen Fluoreszenzfarbstoff aufweisenden fluoreszenz-optischen Sensor (10), der auf den zu untersuchenden Gewebeflächenabschnitt (9) aufgebracht wird,
- mit wenigstens einer Lichtquelle (7) zur Erzeugung von Anregungslicht (13), mit dem der auf dem Gewebeflächenabschnitt (9) aufgebrachte Fluoreszenzfarbstoff zur Erzeugung einer Fluoreszenz beaufschlagt wird,
- mit wenigstens einem Kamerasystems (8) zur Aufnahme zumindest eines ersten Fluoreszenzbildes (FB1) in der Anklingphase (AnP) der Fluoreszenz und zumindest eines zweiten Fluoreszenzbildes (FB2) in der Abklingphase (AbP) der Fluoreszenz,
- mit wenigstens einer Ansteuer- und Auswerteroutine (AAR), mittels der die Fluoreszenzintensitäten und die Intensitätsintegrale (A1, A2) in der An- und Anklingphase (AnP, AbP) anhand der aufgenommenen ersten und zweiten Fluoreszenzbilder (FB1, FB2) bestimmt werden und durch Verhältnisbildung der ermittelten Intenitätsintegrale (A1, A2) die Sauerstoffpar-tialdruckverteilung in dem zumindest einen Gewebeflächenabschnitt (9) ermittelt wird.

**10.** Messsystem nach Anspruch 9, bei dem die Lichtquelle (7) als gepulste Lichtquelle (7) ausgebildet ist und/oder bei dem die Lichtquelle (7) als LED, Blitzlampe oder Laserdiode ausgebildet ist.

**11.** Messsystem nach Anspruch 9 oder 10, bei dem zur Erhöhung der intensität des Anregungslichtes (13) mehrere vorzugsweise als LED ausgebildete Lichtquellen (7) vorgesehen sind.

**12.** Messsystem nach Anspruch 11, bei dem mehrere vorzugsweise als LED ausgebildete Lichtquellen (7) eine Lichtquellengruppe bilden und mehrere derartiger Lichtquellengruppen symmetrisch um das Kamerasystem (8) angeordnet sind.

**13.** Messsystem nach einem der Ansprüche 9 bis 12, bei dem der fluoreszenz-optische Sensor (10) als planarer Sensorfilm oder als Mikropartikelsensor ausgebildet ist und/oder bei dem das Kamerasystem (8) senkrecht über dem zu vermessenden Gewebeflächenabschnitt (9) in einem Abstand (d) zwischen 3 - 15 cm angeordnet ist und/oder bei dem das Kamerasystem (8) zur Aufnahme von Fluuoreszenzbildern (FB1, FB2) und Normalbildern (NB1-NB3) als opto-elektrischer Bildwandler ein Charge-Coupled Duvice (CCD)-Modul (11) aufweist.

**14.** Messsystem nach Anspruch 9 bis 13, bei dem zur Ansteuerung der zumindest einen Lichtquelle (7) sowie des Kamerasystems (8) eine Steuereinheit (3) und eine mit dieser verbundenen Rechnereinheit (3) vorgesehen ist und/oder bei dem die Lichtquelle (7) als gepulste UV-Lichtquelle (7) ausgebildet ist und zusätzlich zu dieser zumindest eine gepulste Normallichtquelle zur Ausleuchtung des Messraumes für die Aufnahme eines Normalbildes (NB1-NB3) vorgesehen ist.

**15.** Messsystem nach Anspruch 14, bei dem die Steuereinheit (3) eine Triggersignaleinheit (3.2) zur Erzeugung eines Triggersignals (ts) aufweist, über welches eine gleichzeitige Ansteuerung der Lichtquelle (7) sowie des Kamerasystems (8) erfolgt.

**Claims**

**1.** Method for determining the oxygen partial pressure distribution in at least one tissue surface section (9), more particularly a skin tissue surface section,

- in which a fluorescent dye is applied,
- in which the fluorescent dye applied to the tissue surface section (9) is charged with an excitation light (13) to generate fluorescence,
- in which in the rising phase (AnP) of the fluorescence at least one first fluorescence picture (FB1) is taken by means of a camera system (8) and in the falling phase (Ab)) of the fluorescence at least one second fluorescence picture (FB2) is taken,
- in which the fluorescence intensities and the intensity integrals (A1, A2) in the rising and falling phases (AnP, AbP) are determined on the basis of the first and second fluorescence pictures (FB1, FB2) taken and

- in which the oxygen partial pressure distribution (P1, P2) in the at least one tissue surface section (9) is ascertained by forming a ratio of the intensity integrals (A1, A2) determined.

2. Method according to claim 1 in which in order to excite the fluorescence pulsed excitation light (13) is used whose spectrum lies preferably within the UV range.

3. Method according to claim 2 in which the pulsed excitation light (13) is generated by means of a pulse light source (7).

4. Method according to one of claims 1 to 3 in which the excitation period is selected equal to the recording period wherein the excitation period preferably comprises 100μs and/or in which the taking of the first and second fluorescence pictures (FB1, FB2) is controlled by a trigger signal (ts) and/or in the rising and falling phases (AnP, AbP) the first and second fluorescence pictures (FB1, FB2) are collected as digital image data and starting from this the fluorescence intensities are determined as the first and second intensity integrals (A1, A2).

5. Method according to one of claims 4 in which the ratio (R) of the first to the second intensity integral (A1, A2) is determined which is a measure for the oxygen partial pressure distribution (P1, P2) present in the tissue surface section (9) which is to be investigated.

6. Method according to one of the preceding claims in which in addition to the fluorescence pictures (FB1, FB2) taken, normal pictures (NB1 - NB3) of the tissue surface section (9) under investigation are taken by the camera system (8) and/or in which porphyrins are used as the fluorescent dye.

7. Method according to claim 6 in which the recording period provided for taking the normal pictures is selected at between 1 and 30 ms.

8. Method according to one of the preceding claims in which prior to applying the fluorescent dye to the tissue surface section (9) under investigation a transparent emulsion or a transparent special fluid is applied and/or in which the intensity of the excitation light (13) is regulated by means of the number of light sources (7) which are preferably designed as LEDs.

9. Measuring system for determining the oxygen partial pressure distribution in at least one tissue surface section (9), in particular a skin tissue surface section,

- with at least one fluorescence-optical sensor (10) having a fluorescent dye and applied to the tissue surface section (9) under investigation,
- with at least one light source (7) for generating excitation light (13) with which the fluorescent dye applied to the tissue surface section (9) is charged in order to generate fluorescence,
- with at least one camera system (8) for taking at least one first fluorescence picture (FB1) in the rising phase (AnP) of the fluorescence and at least one second fluorescence picture (FB2) in the falling phase (AbP) of the fluorescence,
- with at least one control and evaluation routine (AAR) by means of which the fluorescence intensities and the intensity integrals (A1, A2) in the rising and falling phase (AnP, AbP) are determined from the first and second fluorescence pictures (FB1, FB2) taken and by forming a ratio of the determined intensity integrals (A1, A2) the oxygen partial pressure distribution is determined in the at least one tissue surface section (9).

10. Measuring system according to claim 9 in which the light source (7) is designed as a pulsed light source (7) and/or in which the light source (7) is designed as an LED, flash bulb or laser diode.

11. Measuring system according to claim 9 or 10 in which in order to increase the intensity of the excitation light (13) several light sources (7) preferably designed as LEDs are provided.

12. Measuring system according to claim 11 in which several light sources (7) preferably designed as LEDs form one light source group and several such light source groups are arranged symmetrically around the camera system (8).

13. Measuring system according to one of claims 9 to 12 in which the fluorescence-optical sensor (10) is designed as a planar sensor film or as a micro particle sensor and/or in which the camera system (8) is arranged at a distance (d) of between 3 - 15 cm perpendicularly above the tissue surface section (9) under investigation and/or in which the camera system (8) for taking the fluorescence pictures (FB1, FB2) and normal pictures (NB1 - NB3) has a charge-coupled device (CCD) module (11) as opto-electrical image converter.

14. Measuring system according to claims 9 to 13 in which in order to control the at least one light source (7) as well as the camera system (8) a control unit (3) and a computer unit (3) connected to the latter are used and/or in which the light source (7) is formed as a pulsed UV light source (7) and in addition to this at least one pulsed normal light source is provided to illuminate the measuring room for taking a normal picture (NB1-NB3).

15. Measuring system according to claim 14 in which the control unit (3) has a trigger signal unit (3.2) for generating a trigger signal (ts) via which a simultaneous control of the light source (7) and the camera system (8) is carried out.

**Revendications**

1. Procédé pour déterminer la répartition de la pression partielle d'oxygène sur au moins une partie de la surface de tissus (9), notamment sur une partie de la surface de tissus cutanés,

   - dans lequel on applique une substance fluorescente,
   - dans lequel on expose la substance fluorescente appliquée sur la partie de la surface de tissus (9) à une lumière d'excitation (13) pour produire une fluorescence,
   - dans lequel on enregistre au moyen d'un système de caméra (8) pendant la phase d'établissement de la fluorescence (AnP) au moins une première image de fluorescence (FB1) et pendant la phase de décroissance de la fluorescence (AbP) au moins une deuxième image de fluorescence (FB2),
   - dans lequel on détermine les intensités de fluorescence et les intégrales de l'intensité (A1, A2) dans les phases d'établissement et de décroissance (AnP, AbP) à l'aide de la première et de la deuxième images de fluorescence enregistrées (FB1, FB2), et
   - dans lequel on détermine, en formant le rapport des intégrales de l'intensité déterminées (A1, A2), la répartition de la pression partielle d'oxygène (P1, P2) dans ladite au moins une partie de la surface de tissus (9).

2. Procédé selon la revendication 1, dans lequel on utilise, pour l'excitation de la fluorescence, une lumière d'excitation pulsée (13), dont le spectre se situe de préférence dans le domaine UV.

3. Procédé selon la revendication 2, dans lequel on produit la lumière d'excitation pulsée (13) au moyen d'une source de lumière pulsée (7).

4. Procédé selon l'une quelconque des revendications 1 à 3, dans lequel on choisit une durée d'excitation égale à la durée d'enregistrement, la durée d'excitation étant de préférence de 100 μs, et/ou dans lequel on commande l'enregistrement de la première et de la deuxième images de fluorescence (FB1, FB2) au moyen d'un signal de déclenchement (ts) et/ou on enregistre, pendant la phase d'établissement et la phase de décroissance (AnP, AbP), la première et la deuxième images de fluorescence (FB1, FB2) sous la forme de données d'image numériques et on détermine à partir de celles-ci les intensités de fluorescence comme première et deuxième intégrales de l'intensité (A1, A2).

5. Procédé selon la revendication 4, dans lequel on détermine le rapport (R) de la première à la deuxième intégrale de l'intensité (A1, A2), qui est une mesure de la répartition de la pression partielle d'oxygène (P1, P2) présente sur la partie de la surface de tissus (9) à examiner.

6. Procédé selon l'une quelconque des revendications précédentes, dans lequel on enregistre, au moyen du système de caméra (8), des images normales (NB1-NB3) de la partie de la surface de tissus (9) à examiner, en plus des images de fluorescence (FB1, FB2) enregistrées et/ou dans lequel on utilise des porphyrines comme substance fluorescente.

7. Procédé selon la revendication 6, dans lequel on choisit une durée comprise entre 1 et 30 ms comme durée d'enregistrement prévue pour l'enregistrement des images normales.

8. Procédé selon l'une quelconque des revendications précédentes, dans lequel on applique une émulsion transparente ou un fluide spécial transparent sur la partie de la surface de tissus (9) à examiner avant l'application de la substance fluorescente et/ou dans lequel on régule l'intensité de la lumière d'excitation (13) au moyen du nombre des sources de lumière (7) se présentant de préférence sous la forme de DEL.

9. Système de mesure pour déterminer la répartition de la pression partielle d'oxygène sur au moins une partie de la surface de tissus (9), notamment sur une partie de la surface de tissus cutanés,

   - avec au moins un capteur de fluorescence optique (10) présentant une substance fluorescente, qui est appliqué sur la partie de la surface de tissus (9) à examiner,
   - avec au moins une source de lumière (7) pour la production d'une lumière d'excitation (13), à laquelle la substance fluorescente appliquée sur la partie de la surface de tissus (9) est exposée pour produire une fluorescence,
   - avec au moins un système de caméra (8) destiné à enregistrer au moins une première image de fluorescence (FB1) dans la phase d'établissement (AnP) de la fluorescence et au moins une deuxième image de fluorescence (AbP) dans la phase de décroissance de la fluorescence,

- avec au moins un sous-programme de commande et d'évaluation (AAR), au moyen duquel on détermine les intensités de fluorescence et les intégrales de l'intensité (A1, A2) dans la phase d'établissement et de décroissance (AnP, AbP) à l'aide de la première et de la deuxième images de fluorescence enregistrées (FB1, FB2) et on détermine la répartition de la pression partielle d'oxygène dans ladite au moins une partie de la surface de tissus (9) en formant le rapport des intégrales de l'intensité déterminées (A1, A2).

10. Système de mesure selon la revendication 9, dans lequel la source de lumière (7) se présente sous la forme d'une source de lumière pulsée (7) et/ou dans lequel la source de lumière (7) se présente sous la forme de DEL, d'une lampe éclair ou d'une diode laser.

11. Système de mesure selon la revendication 9 ou 10, dans lequel il est prévu plusieurs sources de lumière (7) se présentant de préférence sous la forme de DEL pour augmenter l'intensité de la lumière d'excitation (13).

12. Système de mesure selon la revendication 11, dans lequel plusieurs sources de lumière (7) se présentant de préférence sous la forme de DEL forment un groupe de sources de lumière et plusieurs de tels groupes de sources de lumière sont disposés symétriquement autour du système de caméra (8).

13. Système de mesure selon l'une quelconque des revendications 9 à 12, dans lequel le capteur à fluorescence optique (10) se présente sous la forme d'un capteur à film plan ou d'un capteur à microparticules t/ou dans lequel le système de caméra (8) est disposé à une distance (d) comprise entre 3 et 15 cm perpendiculairement au-dessus de la partie de la surface de tissus (9) à mesurer et/ou dans lequel le système de caméra (8) présente un module de dispositif à couplage de charge (CCD) comme convertisseur d'image électro-optique pour l'enregistrement d'images de fluorescence (FB1, FB2) et d'images normales (NB1-NB3).

14. Système de mesure selon l'une quelconque des revendications 9 à 13, dans lequel il est prévu une unité de commande (3) et une unité d'ordinateur (3) reliée à celle-ci pour commander ladite au moins une source de lumière (7) ainsi que le système de caméra (8) et/ou dans lequel la source de lumière (7) se présente sous la forme d'une source de lumière UV pulsée (7) et il est prévu en plus de celle-ci au moins une source de lumière normale pulsée pour l'éclairage de la chambre de mesure en vue de l'enregistrement d'une image normale (NB1-NB3).

15. Système de mesure selon la revendication 14, dans lequel l'unité de commande (3) comprend une unité de signal de déclenchement (3.2) pour produire un signal de déclenchement (ts), au moyen duquel il se produit une commande simultanée de la source de lumière (7) ainsi que du système de caméra (8).

Fig. 1

Fig. 2

12

EP 1 910 808 B1

Fig. 3

a)

b)

$R = A1 / A2$

Sauerstoff

R

Fig. 4

a)

b)

Fig. 5

a)

b)

Fig. 6

16

15

I1

PP1

Fig. 7

16

15

I2

PP2

Fig. 8

16

15

VV

PP3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10157575 A1
- US 5593899 A
- US 20030050543 A1
- US 6345191 B1
- US 4476870 A